# EUROPÄISCHE PATENTSCHRIFT

(11) **EP 0 550 523 B1**
(45) Veröffentlichungstag und Bekanntmachung des Hinweises auf die Patenterteilung: **02.11.1994**
(21) Anmeldenummer: 91916759.3
(22) Anmeldetag: 24.09.1991
(51) Int. Cl.: G01N 1/20

(54) **VERFAHREN UND VORRICHTUNG ZUR PROBENGEWINNUNG UND PROBENHANDHABUNG**
PROCESS AND DEVICE FOR OBTAINING AND HANDLING SAMPLES
PROCEDE ET DISPOSITIF POUR L'EXTRACTION ET LA MANUTENTION D'ECHANTILLONS

(30) Priorität: 28.09.1990 DE 4030729
(43) Veröffentlichungstag der Anmeldung: 14.07.1993
(73) Patentinhaber: OTTO TUCHENHAGEN GmbH & Co. KG, 21514 Büchen (DE)
(72) Erfinder: RIESS, Siegfried, D-2059 Güster (DE); EVERS, Klaus, Detlef, Marcus, D-2059 Büchen (DE)
(74) Vertreter: Glaeser, Joachim, Dipl.-Ing.
(86) Internationale Anmeldenummer: EP9101817
(87) Internationale Veröffentlichungsnummer: WO9206363

(56) Entgegenhaltungen:
- EP-A- 0 033 709
- WO-A-88/00234
- GB-A- 2 162 647
- US-A- 3 473 388
- US-A- 4 532 813
- US-A- 4 873 876

## Beschreibung

Die Erfindung betrifft ein Verfahren zur Probengewinnung und Probenhandhabung, insbesondere für Flüssigkeiten mit unter Druck gelösten Gasen im Bereich der Nahrungsmittel- und Getränkeindustrie, bei dem jeweils während der Überführung eines zu prüfenden Flüssigkeitsvolumens in einer Durchflußleitung ein dem überführten Gesamtvolumen repräsentatives Probenvolumen in vom Staudruck abhängiger Menge fortlaufend aus der Durchflußleitung in eine Probenflasche abgezweigt wird, wobei der statische Druck im abgezweigten Volumen dem statischen Druck an der Entnahmestelle der Durchflußleitung durch Regelung mittels einer Fremdgasquelle angeglichen wird, und eine Vorrichtung zur Durchführung des Verfahrens.

Aus der GB-A2 162 647 ist ein Verfahren zur Gewinnung eines proportionalen Teilstromes eines in einer Durchflußleitung fließenden Fluids bekannt, bei dem aus der Durchflußleitung in vom Staudruck abhängiger Menge fortlaufend ein Teilvolumen in ein Gefäß abgezweigt wird, und bei dem der statische Druck im abgezweigten Volumen dem statischen Druck an der Entnahmestelle über eine Fremddruckquelle angeglichen wird. Das bekannte Verfahren und die Vorrichtung zu seiner Durchführung tragen der physikalischen Erkenntnis Rechnung, daß eine repräsentative Probennahme nur dann möglich ist, wenn Druckgleichheit zwischen dem statischen Druck an der Entnahmestelle und im abgezweigten Volumen herrscht. Der Druckschrift ist allerdings nicht zu entnehmen, wie die Angleichung des statischen Druckes des im Gefäß abgezweigten Volumens mit jenem an der Entnahmestelle der Durchflußleitung praktisch durchgeführt werden soll und welche Größe konkret als Stellgröße für den Druckausgleich vorgesehen ist.

Aus der US-A-45 32 813 ist darüber hinaus eine Vorrichtung zur Gewinnung von Proben aus einer strömenden Flüssigkeit bekannt, bei der das Probengefäß zwar auf Überdruck gehalten, jedoch auf einen Druck, der niedriger als derjenige in einem vorgeschalteten Sammelgefäß ist. Dadurch soll der Fluß vom Sammel- zum Probengefäß gewährleistet sein. Die bekannte Vorrichtung beinhaltet in der Leitung, die die zu probende Flüssigkeit führt, eine Einrichtung zur Durchflußmengenmessung, wobei die Durchflußmessung zur Ansteuerung eines Ventils dient. In Abhängigkeit von der Durchflußmenge wird die Öffnungszeit dieses Ventils gewählt, um die gewünschte Flüssigkeitsmenge zur Bildung einer repräsentativen Probenmenge in das Sammelgefäß zu leiten. Mit der bekannten Probenahme-Vorrichtung ist es nicht möglich, Druckschwankungen in der die zu probende Flüssigkeit führenden Durchflußleitung im Innenraum des Probengefäßes auszugleichen. Druckschwankungen in der Durchflußleitung führen daher zu einer Verfälschung der Probe; eine streng mengenproportionale Teilstromentnahme ist mit der bekannten Vorrichtung daher nicht möglich.

Des weiteren ist aus dem deutschen Gebrauchsmuster 85 25 252 eine Vorrichtung zur unmittelbaren Überführung von Milchteilmengen aus einer Milchförderleitung in eine Probenflasche bekannt, bei der die Probenflasche über einen elastischen Verschlußstopfen oder eine elastische Verschlußmembran form- und/oder kraftschlüssig an einer peripheren Öffnung der Milchförderleitung dichtend befestigt ist und bei der eine Füll- und eine Entlüftungsnadel vorgesehen sind, die in ihrer axialen Richtung gemeinsam bewegbar und dabei in der Probenentnahmestellung einerseits in die Probenflasche verbringbar sind und andererseits sowohl in der Probenentnahme- als auch in der Spülstellung mit einem Abstand voneinander in den Innenraum der Milchförderleitung ausmünden. In diesem Zusammenhang wird nun vorgeschlagen, eine Füll- oder eine Entlüftungsbohrung, die in den Innenraum der Milchförderleitung ausmündet und der Füll- bzw. Entlüftungsnadel zugeordnet ist, in ihrer Probenentnahmestellung mittels eines über eine Hubeinrichtung betätigbaren Schließgliedes intermittierend zu verschließen. Bei richtiger Anordnung der Füll- und der Entlüftungsbohrung innerhalb der Durchflußleitung gewährleistet diese bekannte Vorrichtung eine Probenentnahme in vom Staudruck abhängiger Menge, wodurch die Repräsentativität der gewonnenen Probe in aller Regel sichergestellt ist.

Obgleich die Probenflasche mit einem elastischen Verschlußstopfen oder einer elastischen Verschlußmembran verschlossen ist, wobei sich diese Verschlußmittel nach dem Herausziehen der Injektionsnadeln wieder flüssigkeitsdicht selbst schließen, sind mit der bekannten Vorrichtung keine Flüssigkeitsproben zu gewinnen, bei denen nach dem Entfernen der Probenflasche von der Anschlußstelle an der Durchflußleitung dauerhaft der statische Druck, wie er in der Durchflußleitung zum Zeitpunkt der Probenentnahme vorlag, erhalten bleibt. Dies resultiert aus der Tatsache, daß die vorgenannten Verschlußmittel zwar gegenüber Flüssigkeit selbstdichtende Eigenschaften besitzen, daß sie jedoch für Gas, welches unter einem Druck in der Probenflasche ansteht, der höher oder niedriger als der Umgebungsdruck ist, durchlässig sind. Die bekannte Vorrichtung erlaubt zwar die Probengewinnung und -überführung unter dem statischen Druck, wie er in der Durchflußleitung vorliegt; die Probenaufbewahrung in der und die Entnahme aus der Probenflasche ist jedoch bei diesem statischen Druck dauerhaft nicht mehr sichergestellt. Die Folge dieser zwangsläufigen Druckabsenkung ist dann, falls Flüssigkeiten mit unter Druck gelösten Gasen, wie beispielsweise Bier oder andere kohlensäurehaltigen Getränke, einer Probennahme unterzogen werden sollen, daß die gelösten Gase aus der Flüssigkeit entbinden und daß sich dadurch die Eigenschaft der Flüssigkeitsprobe gegenüber ihrem Zustand zum Zeitpunkt ihrer Gewinnung verändert. Probenflaschen mit elastischen Verschlußmitteln, die von Kanülen durchstochen und durch diese mit Probenflüssigkeit beaufschlagt werden, sind beispielsweise auch aus der EP 0 319 072 A1 und DE 29 07 558 A1 bekannt.

Im Zusammenhang mit der isobarometrischen Gewinnung der Probe, ihrer Aufbewahrung und ihrer Entnahme aus der Probenflasche wird, inbesondere im Bereich der Nahrungsmittel- und Getränkeindustrie, darüber hinaus die Forderung gestellt, daß die Probengewinnung und -handhabung unter sterilen und bakteriologisch einwandfreien Bedingungen und oftmals noch zusätzlich unter Ausschluß schädlichen Luftsauerstoffes zu erfolgen hat. Von der Gewinnung der Probe vor Ort aus der Durchflußleitung bis zu ihrer Analyse im Labor ist ihre Handhabung unter diesen Bedingungen lückenlos sicherzustellen. Keine im Bereich der Nahrungsmittel- und Getränkeindustrie bislang bekannt gewordene Probennahmevorrichtung, welche eine Probe unter isobarometrischen Bedingungen aus einer Durchflußleitung gewinnt, schafft mit den hierfür vorgesehenen Probenflaschen die Voraussetzungen dafür, daß auch die anderen, im Anschluß an die Probennahme durchzuführenden Verfahrensschritte bis hin zur Probenanalyse unter den geforderten Bedingungen durchgeführt werden können.

Die vorgenannten sterilen Bedingungen lassen sich praktisch nur dadurch realisieren, daß nicht ortsfeste Teile der Probennahmevorrichtung, beispielsweise die Probenflaschen und ein bestimmtes Zubehör, bei relativ hohen Temperaturen sterilisierbar bzw. autoklavierbar sind. Andere Teile der Probennahmevorrichtung, die aufgrund ihrer ortsfesten Anordnung an der Durchflußleitung nicht in der vorstehend angegebenen Weise behandelt werden können, müssen zumindest reinigungsfreundlich ausgestaltet werden und im Bereich, der mit Probenflüssigkeit kontaminiert wird, auf dynamisch beanspruchte Dichtungen bzw. auf Dichtungen allgemein verzichten.

Der Erfindung liegt die Aufgabe zugrunde, ein Verfahren und eine Vorrichtung zu seiner Durchführung zu schaffen, mit denen eine repräsentative Probennahme aus einer Durchflußleitung und eine Probenüberführung in eine, eine Probenaufbewahrung in der und eine Probenentnahme aus der Probenflasche unter sterilen und isobarometrischen Bedingungen und erforderlichenfalls unter Ausschluß schädlichen Luftsauerstoffes möglich ist.

Diese Aufgabe wird durch Anwendung der Kennzeichenmerkmale des Anspruchs 1 gelöst. Vorteilhafte Ausgestaltungen des erfindungsgemäßen Verfahrens sind Gegenstand der Ansprüche 2 bis 8. Eine Vorrichtung zur Durchführung des erfindungsgemäßen Verfahrens ergibt sich durch die Ausgestaltung mit den Kennzeichenmerkmalen des Anspruches 9, während vorteilhafte Ausführungsformen der erfindungsgemäßen Vorrichtung Gegenstand der Unteransprüche 10 bis 18 sind.

Die Vorteile des erfindungsgemäßen Verfahrens und der Vorrichtung zu ihrer Durchführung sind augenfällig. Die leeren, zur Befüllung anstehenden Probenflaschen werden im geschlossenen, druckdichten Zustand bereitgestellt, wobei es sich bei der Probenflasche beispielsweise um eine genormte Glasflasche mit Schraubgewinde am Flaschenhals handeln kann, die auch in druckfester Ausführung im Handel erhältlich sind. Eine zumindest in ihrer prinzipiellen Wirkungsweise an sich bekannte Verschlußvorrichtung, wie sie beispielsweise bei Bierfässern Anwendung findet (US-PS 37 76 260), ist als Probenflaschenverschluß vorgesehen, wobei im Zuge des Verbindungsvorganges der druckdicht verschlossenen Probenflasche mit einer Probenflaschenkupplung ein erster Verbindungsweg zwischen dem Innenraum der Probenflasche und einer Probenabzweiganordnung der Durchflußleitung und ein zweiter Verbindungsweg zwischen dem Innenraum der Probenflasche und einer Druckausgleichsleitung geöffnet werden und wobei, einer vorteilhaften Ausgestaltung der Vorrichtung, zur Durchführung des Verfahrens gemäß der Erfindung, der erste Verbindungsweg im Bodenbereich und der zweite im im Kopfbereich der Probenflasche ausmünden. Die Verschlußvorrichtung und die Probenflaschenkupplung sind beispielsweise sterilisierbar bzw. autoklavierbar bis zu einer Temperatur von 150 oC und die Verschlußvorrichtung wird zweckmäßigerweise bis zu ihrer mittels der Probenflaschenkupplung hergestellten Verbindung mit der Probenabzweiganordnung mit einer sterilen Abdeckkappe versehen. Die mit Probenflüssigkeit kontaminierten Oberflächen der Probenabzweiganordnung können vor jeder Probenentnahme ebenfalls gereinigt und sterilisiert und ihre aus der Umgebung direkt zugänglichen diesbezüglichen Oberflächen können vor dem Anschluß der Probenflaschenkupplung mit einer sterilen Abdeckkappe versehen werden.

Durch die vorgeschlagene Vorrichtung und durch das durch sie durchführbare Verfahren mit seinen vorgesehenen Ausgestaltungen ist es möglich, zunächst, gemäß einer ersten Ausgestaltung, den Innenraum der Probenflasche mit Inertgas zu durchspülen und dadurch ggf. die in ihr enthaltene Luft zu entfernen und durch dieses Gas zu substituieren, so daß die anschließende Probenentnahme unter Ausschluß der in aller Regel schädlichen Luft bzw. des Luftsauerstoffes stattfinden kann. Darüber hinaus ist auch, gemäß einer weiteren Ausgestaltung des vorgenannten Verfahrens, ein Vorspannen der Probenflasche mit dem Inertgas auf den statischen Druck an der Entnahmestelle der Durchflußleitung möglich, wodurch eine isobarometrische Probenentnahme bereits vom ersten Augenblick der Probenüberführung in die Probenflasche gewährleistet ist.

Gemäß einer vorteilhaften Ausgestaltung des Verfahrens ist vorgesehen, die Probenflasche, falls erforderlich, mit einer Nährlösung oder mit einem anderen für analytische Zwecke notwendigen Substrat bzw. mit einer Lösung vorzufüllen, die Probenflasche und ihr unter Abschluß stehender Inhalt gemeinsam zu sterilisieren und das Probenvolumen im Zuge der Probengewinnung in die derart vorbehandelte Probenflasche einzubringen.

Die Gleichhaltung des statischen Druckes an der Entnahmestelle der Durchflußleitung und im Innenraum der Probenflasche erfolgt geregelt mittels einer Fremdgasquelle, die mit einem Gleichdruckregler verbunden ist, der durch den Druck der Durchflußleitung an der Entnahmestelle beaufschlagt wird, so daß auch bei schwankendem Druck in der Durchflußleitung eine repräsentative Probenentnahme sichergestellt ist.

Ein allein vom Staudruck der Strömung in der Durchflußleitung abhängiger Probenvolumenstrom ist, streng genommen, nur möglich, wenn keine geodätischen Höhendifferenzen zwischen Entnahme- und Austrittsstelle des Probenstromes in Kauf zu nehmen wären und ein absolut trägheitsfrei arbeitender Gleichdruckregler verfügbar wäre. Da derartige ideale Bedingungen prinzipiell nicht realisierbar sind, wird bei der vorgeschlagenen Vorrichtung durch hinreichend große Fließgeschwindigkeit im Bereich der Entnahmestelle in der Durchflußleitung dafür Sorge getragen, daß der hinzunehmende Fehler ein zulässiges Maß nicht überschreitet. Dies ist dann der Fall, wenn, wie dies eine vorteilhafte Ausgestaltung des Verfahrens vorsieht, der Staudruck an der Entnahmestelle die hinzunehmende statische Druckdifferenz zwischen Entnahme- und Austrittsstelle um mindestens das fünffache übersteigt.

Mit der vorgeschlagenen Vorrichtung zur Durchführung des Verfahrens ist nicht nur eine Probenentnahme möglich, bei der der Druck in der Durchflußleitung über jenem in der Umgebung der Vorrichtung liegt, sondern auch eine Probenentnahme unter Vakuum, da die notwendige Druckdifferenz zum Transport des aus der Durchflußleitung abgezweigten Teilstromes in die Probenflasche durch den Staudruck der Strömung in der Durchflußleitung bereitgestellt wird.

Während der Loslösung der Probenflasche von der Probenabzweiganordnung nach Beendung der Probenentnahme wird die Probenflasche über die Verschlußvorrichtung druckdicht verschlossen, so daß sich die Flüssigkeitsprobe nunmehr unter dem statischen Druck, der bei der Probenentnahme herrschte, befindet und dauerhaft verbleibt. Dies wird dadurch sichergestellt, daß die Verschlußvorrichtung der Probenflasche mit einer federschließenden Dichtungsanordnung versehen ist, die hinsichtlich der wirkenden Schließkraft durch den statischen Druck in der Probenflasche unterstützt wird.

Die Probenflasche kann in Verbindung mit dem Verschlußteil aus ihrem Bodenbereich heraus entleert werden, so daß sichergestellt ist, daß die Probenflüssigkeit unter gleichen isobarometrischen und sterilen Bedingungen aus der Probenflasche entfernt und einem Analysengerät zugeleitet werden kann, wie dies beim Einleiten der Probenflüssigkeit in die Probenflasche im Zuge der Probenentnahme gewährleistet war.

Das erfindungsgemäße Verfahren sieht weiterhin eine getaktete oder eine kontinuierliche Abzweigung des Probenvolumens aus der Durchflußleitung in die Probenflasche vor, wobei die jeweilige Verfahrensweise durch entsprechende Ansteuerung eines Ventils bestimmt wird und die Größe des abgezweigten Probenvolumenstromes sich einerseits entweder aus der Öffnungszeit des Ventils (≧ 0,1 Sekunde) und der Taktfolge (≧ 1 Sekunde) oder aus einem wahlweise veränderbaren Hub des Ventils oder andererseits aus wahlweise veränderbaren Abmessungen des Durchtrittsquerschnittes in einem Abzweigrohr oder einer Zulaufleitung im Bereich zwischen dem Abzweigrohr und dem Ventil ergibt.

Durch zeitliches Takten und unterschiedliche Bemessung des abzuzweigenden Probenvolumens einerseits und die Verwendung unterschiedlich großer Probenflaschen bzw. anderer Probengefäße andererseits läßt sich die Dauer der Probenentnahme über einen großen zeitlichen Bereich variieren, wodurch eine Anpassung der Probenentnahme an unterschiedliche Betriebsbedingungen, Volumenströme und damit in der Durchflußleitung transportierte Gesamtvolumina möglich ist.

Zusammengefaßt ergibt sich, daß das vorgeschlagene Verfahren zur Probengewinnung und Probenhandhabung eine sterile, isobarometrische Probennahme gewährleistet. Das Verfahren ist insbesondere zur biologischen Kontrolle länger dauernder Prozeßabläufe in Getränke- und Lebensmittelbetrieben, z. B. bei der Filtration und Abfüllung von Bier, bei der stationären Annahme von Milch oder zur Probennahme in der Non-Food-Industrie geeignet. Hierbei hat sich gezeigt, daß die Verkeimung der gewonnenen Probe repräsentativ ist der durchschnittlichen Verkeimung des Prozeßmediums über die Produktionszeit. Auch bei quasikontinuierlichem Probenabzweig durch Taktung des Probenabzweigventils wird mit hinreichend großer Treffsicherheit jede temporäre Verkeimung erfaßt.

Um eine Überfüllung der Probenflasche zu verhindern, sieht eine Ausgestaltung des erfindungsgemäßen Verfahrens vor, daß der Befüllungsgrad der Probenflasche mittels eines Flüssigkeit von Gas unterscheidenden Signalgebers erfaßt und die Probengewinnung durch ein diesbezügliches Signal unverzüglich unterbrochen wird.

Damit bei Bruch oder Leckage der Probenflasche oder einer Leckage der Probenabzweiganordnung eine weitere Probenentnahme unterbunden werden kann, sieht eine andere Ausgestaltung des erfindungsgemäßen Verfahrens vor, den mit dem Eintritt des vorgenannten Ereignisses verbundenen Druckabfallzu erfassen und mit einem diesbezüglichen Signal die Probengewinnung unverzüglich zu unterbrechen.

Für den Fall, daß der Druck in der Durchflußleitung zusammenbricht, weil beispielsweise diese Leitung unvorhergesehen leer läuft, wird gemäß einer weiteren Ausgestaltung des erfindungsgemäßen Verfahrens dieser Druckabfall erfaßt und ein diesbezügliches Signal wird zur Schließung der beiden Verbindungswege zur Probenflasche herangezogen.

Die Vorrichtung zur Durchführung des erfindungsgemäßen Verfahrens ist, wie vorstehend bereits dargelegt, im Bereich der Probenflasche und der Probenflaschenkupplung bei hohen Temperaturen sterilisierbar bzw. autoklavierbar und in allen relevanten Bereichen der Probenabzweiganordnung voll reinigungsfähig und dämpfbar, wobei die Reinigungsflüssigkeit aus der Durchflußleitung herangeführt und über den Weg, den die Probenflüssigkeit nimmt, durch die Probenabzweiganordnung hindurchgeleitet wird. Die gute Reinigungsfähigkeit der Probenabzweiganordnung resultiert aus der Tatsache, daß sie in ihrem mit Probenflüssigkeit kontaminierten Bereich über keine Dichtungen verfügt.

Eine vorteilhafte Ausgestaltung der Vorrichtung zur Durchführung des erfindungsgemäßen Verfahrens sieht vor, daß die Probenabzweiganordnung einen Ventilblock aufweist, in dem wesentliche, zur Probenentnahme notwendige Funktionen zusammengefaßt sind. Zum einen sind an dem Ventilblock die drei Ventile angeflanscht, die in ihrem grundsätzlichen Aufbau aus der Druckschrift W090/12972 bekannt sind. Die Schließglieder der Ventile greifen von außen in den Ventilblock hinein und wirken dort jeweils mit einer in diesem ausgebildeten Sitzfläche zusammen. Insoweit hat das bekannte Probenventil eine Abwandlung erfahren. In der bekannten Ausführung ist die Sitzfläche im Gehäuse des Probenventils ausgebildet. Auf dieses Gehäuse wird bei der vorgeschlagenen Vorrichtung verzichtet. Es wird lediglich auf den Antrieb, das Schließglied und das letzteres haubenförmig umschließende, in Schließrichtung dehnbare Dichtungsteil zurückgegriffen. Das mit dem Schließglied und dem Dichtungsteil korrespondierende Gehäuse findet seine Realisierung in dem Ventilblock. Darüber hinaus dient der Ventilblock der Aufnahme jeweils eines Abschnittes des zweiten Leitungsabschnittes der Gasleitung, eines Abschnittes der Druckausgleichsleitung, der Zulaufleitung, der Auslaßleitung, des ersten Leitungsabschnittes der Gasleitung und des in die Durchflußleitung hineinragenden Abzweigrohres.

Die Anordnung wesentlicher funktionsrelevanter Vorkehrungen in einem einzigen Bauteil stellt einerseits überhaupt erst die sterile und biologisch einwandfreie Handhabung der Probe sicher, da anderenfalls eine Vielzahl von Dichtungen notwendig wäre, und andererseits erlaubt diese Ausgestaltung erst die Verwendung der bekannten Probennahmenventile in der vorgeschlagenen Weise.

Gemäß einer vorteilhaften Ausführungsform wird sowohl in der Probenabzweiganordnung und hier insbesondere im Ventilblock als auch in der Probenflaschenkupplung in allen mit Probenflüssigkeit kontaminierten Bereichen auf jegliche Dichtungen verzichtet. Lediglich das das Schließglied haubenförmig umschließende Dichtungsteil ist mit seinem Rand im Ventilblock eingespannt. Hierbei handelt es sich um eine statisch beanspruchte Dichtung, die die Funktion einer Gehäusedichtung hat und reinigungstechnisch unbedenklich ist.

Gemäß einer weiteren Ausgestaltung der erfindungsgemäßen Vorrichtung ist die Probenflaschenkupplung als ein von der Probenabzweiganordnung getrenntes, separates Bauteil ausgebildet. Hierdurch wird erreicht, daß wesentliche Teile der Vorrichtung, nämlich jene Teile, die zur druckdichten Verbindung der Probenflasche mit der Probenabzweiganordnung notwendig sind, nicht mehr ortsfest an der Durchflußleitung verbleiben müssen, sondern in geeigneten Einrichtungen bei höheren Temperaturen sterilisiert bzw. autoklaviert werden können.

Die Verbindung zwischen der Probenabzweiganordnung und der Probenflaschenkupplung erfolgt, wie dies eine andere Ausgestaltung der Vorrichtung vorsieht, durch leicht lösbare und ebenfalls problemlos sterilsierbare bzw. autoklavierbare Schlauchverbindungen, welche durch Schlauchabsperrmittel, wie beispeilsweise Schlauchklemmen, abgesperrt werden können, damit bei der Loslösung der Probenflasche vom Ventilblock verhindert wird, daß über die auf die Probenflasche aufgesetzte Probenflaschenkupplung aus der noch geöffneten Probenflasche Druck oder Flüssigkeit entweichen.

Die erfindungsgemäße Vorrichtung ist gemäß einer weiteren Ausführungsform so bemessen und ausgestaltet, daß sie in einer einzigen größenmäßigen Demensionierung für alle in der Praxis vorkommenden Nennweiten der Durchflußleitungen geeignet ist.

Das erfindungsgemäße Verfahren und die Vorrichtung zu seiner Durchführung werden anhand eines Ausführungsbeispiels in den nachfolgend erläuterten Figuren der Zeichnungen im einzelnen näher beschrieben.
Es zeigen
- Figur 1: in schematischer Darstellung eine erfindungsgemäße Vorrichtung in Verbindung mit einer Probenflasche, wobei vorrangig die funktionellen Zusammenhänge dargestellt sind;
- Figur 2: die erfindungsgemäße Vorrichtung gemäß Figur 1 in weitgehend konkreter Ausgestaltung, soweit es die Probenabzweiganorndung und die Probenflasche in Verbindung mit der Probenflaschenkupplung betrifft;
- Figuren 3: einen Mittelschnitt durch den Kopfbereich einer Probenflasche im Zusammenwirken mit einer Verschlußvorrichtung;
- Figur 4: einen Mittelschnitt durch eine Probenflaschenkupplung in ihrer Verbindung mit der Verschlußvorrichtung gemäß Figur 3 und
- Figur 5: einen Mittelschnitt durch die bevorzugte Ausführungsform der Probenabzweiganordnung gemäß Figur 2 in ihrer Verbindung mit der Druchflußleitung, wobei der Ventilblock und ein Ventil gegenüber ihrer Darstellung in Figur 2 detaillierter bezeichnet sind.

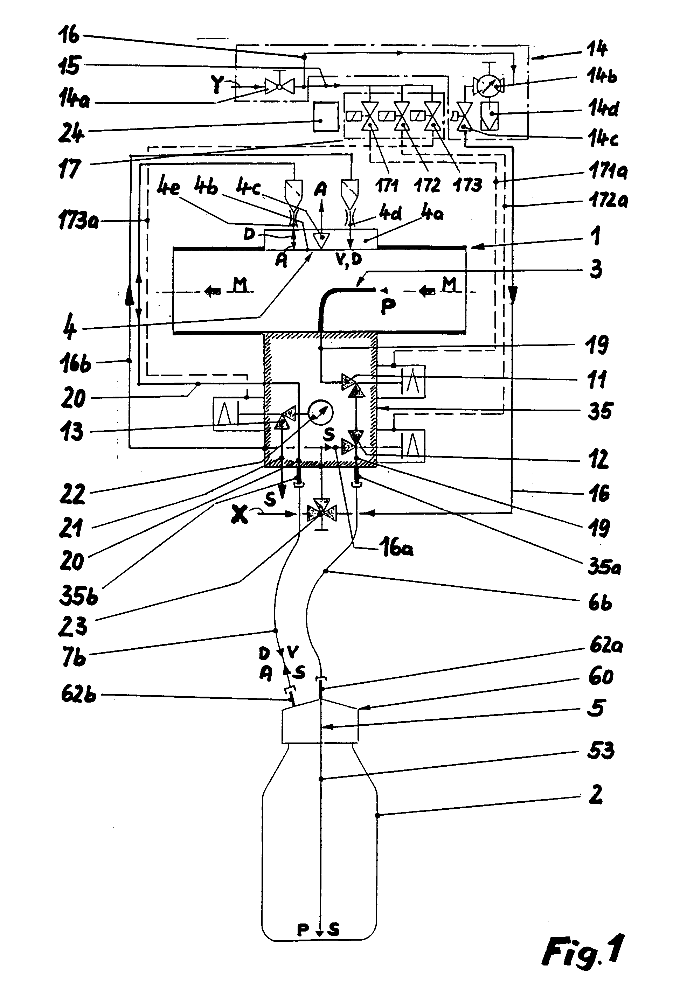

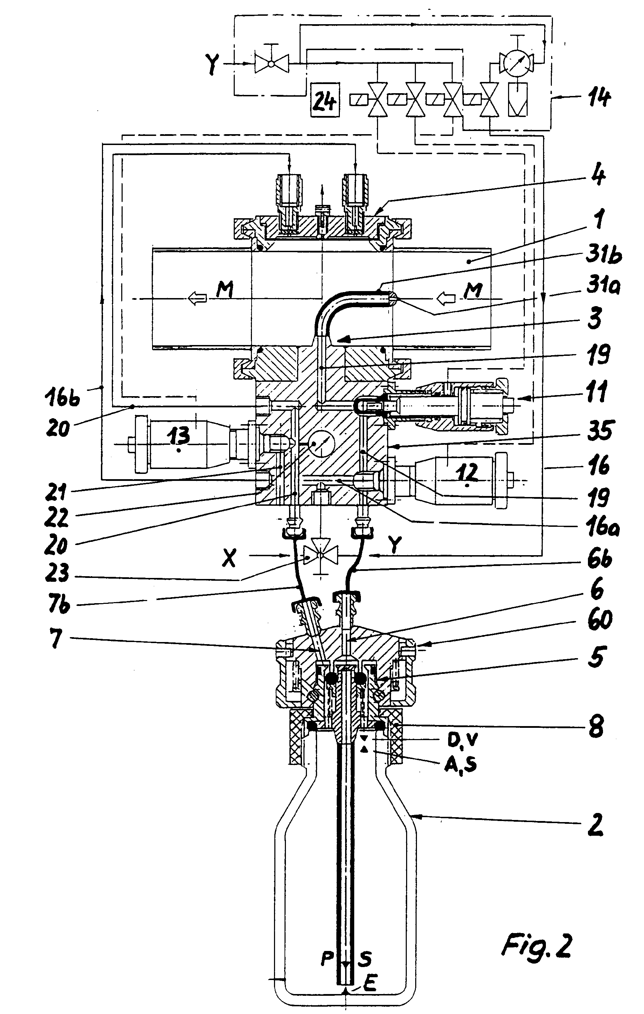

Der prinzipielle Aufbau und die Wirkungsweise der Vorrichtung seien anhand der wesentlichen Bauteile, wie sie in Figur 1 schematisch dargestellt sind, erläutert. Eine Durchflußleitung 1 werde in Pfeilrichtung M vom Prozeßmedium durchströmt, wobei ein Teilstrom P aus dem Gesamtstrom über eine Probenabzweiganordnung 3 in eine darunter angeordnete Probenflasche 2 abgezweigt wird. Diese Abzweigung erfolgt über eine Zulaufleitung 19, die über ein erstes Ventil 11 gesteuert wird, über einen nicht bezeichneten ersten Verbindungsweg 6 im Bereich einer Verschlußvorrichtung 5 der Probenflasche 2 (siehe hierzu Figur 2) und über ein Probenabzweigrohr 53, welches im Bodenbereich der Probenflasche 2 endet. Eine Druckausgleichsleitung 20 verbindet über einen ebenfalls nicht bezeichneten zweiten Verbindungsweg 7 im Bereich der Verschlußvorrichtung 5 (siehe hierzu Figur 2) den Kopfraum der Probenflasche 2 mit einem Anschluß 4e eines Gleichdruckreglers 4, welcher über eine Membran 4b durch den Druck des Prozeßmediums M in der Durchflußleitung 1 an der Entnahmestelle beaufschlagt wird. Der Gleichdruckregler 4 weist darüber hinaus zwei weitere Anschlüße 4c und 4d auf, wobei der Anschluß 4d als Druckmitteleintritt ausgebildet und mit einem zweiten Leitungsabschnitt 16b einer zweiten Gasleitung 16 verbunden ist. Der Anschluß 4c stellt eine Abblasbohrung dar, über die der Innenraum eines Membrangehäuses 4a mit der Umgebung des Gleichdruckreglers 4 bzw. der Vorrichtung zur Probengewinnung und Probenhandhabung verbunden ist.

Die zweite Gasleitung 16 verzweigt sich in zwei Leitungsabschnitte, wobei der zweite Leitungsabschnitt 16b, wie vorstehend bereits erwähnt, mit dem Gleichdruckregler 4 verbunden ist, und ein durch ein zweites Ventil 12 gesteuerter erster Leitungsabschnitt 16a zwischen dem ersten Ventil 11 und einer Probenflaschenkupplung 60 in die Zulaufleitung 19 einmündet. Die Druckausgleichsleitung 20 ist über eine durch ein drittes Ventil 13 gesteuerte Auslaßleitung 21 mit der Umgebung der Vorrichtung verbunden.

Die zweite Gasleitung 16 ist mit einer Fremdgasquelle 14 verbunden und wird aus dieser mit Inertgas Y gespeist. Hierzu sind innerhalb der Fremdgasquelle 14 ein erstes Absperrventil 14a, ein Reduzierventil 14b, ein Pilotventil 14c und ein Filter 14d vorgesehen. Die zweite Gasleitung 16 ist über ein Zweiwege-Umschaltventil 23 geführt, welchem über eine nicht näher bezeichnete Leitung Dampf X zugeführt werden kann.
Abhängig von der jeweiligen Stellung des Zweiwege-Umschaltventils 23 kann der sich in die Leitungsabschnitte 16a und 16b verzweigenden Gasleitung 16 entweder Inertgas Y von der Fremdgasquelle 14 oder aber Dampf X zugeführt werden. Hinter dem ersten Absperrventil 14a verzweigt sich die zweite Gasleitung 16 in eine erste Gasleitung 15, über die im Druck nicht reduziertes Inertgas Y die Antriebszylinder der Ventile 11, 12 und 13 mit Druckmittel versorgt. Hierzu dient eine Ansteuerung 17, in der sich die Pilotventile 171, 172 und 173 befinden, welche über eine erste bzw. eine zweite bzw. eine dritte Ansteuerungsleitung 171a bzw. 172a bzw. 173a mit den nicht näher bezeichneten Antriebszylindern der Ventile 11 bzw. 12 bzw. 13 verbunden sind. Bei den Pilotventilen handelt sich um 3/2 Wegeventile, wobei die Anschlüsse, über die das Druckmittel aus den Antriebszylindern in die Umgebung abgeführt wird, in einer nicht dargestellten Ablaßleitung zusammengeführt sind. Mit 24 ist eine Steuerung bezeichnet, die die erfindungsgemäße Vorrichtung im Rahmen der vorgesehenen Verfahrensschritte planmäßig steuert.

Die Probenabzweiganordnung 3 weist einen Ventilblock 35 auf, der der Aufnahme der Ventile 11, 12 und 13 dient. Darüber hinaus sind in ihm ein Abschnitt des zweiten Leitungsabschnittes 16b der Gasleitung 16, ein Abschnitt der Druckausgleichsleitung 20, die Zulaufleitung 19, die Auslaßleitung 21, der erste Leitungsabschnitt 16a der Gasleitung 16 und ein in die Durchflußleitung 1 hineinragendes Abzweigrohr 31b (siehe hierzu Figur 2) angeordnet. Die Zulaufleitung 19 und die Druckausgleichsleitung 20 weisen an ihrem der Probenflaschenkupplung 60 zugewandten Ende jeweils einen Schlauchanschluß 35a bzw. 35b auf. An der Probenflaschenkupplung 60, die als ein von der Probenabzweiganordnung 3 getrenntes, separates Bauteile ausgebildet ist, sind ebenfalls zwei Schlauchanschlüsse 62a und 62b angeordnet, von denen der erste zum ersten Verbindungsweg 6 und der zweite zum zweiten Verbindungsweg 7 führt (vgl. hierzu Figur 2). Die Schlauchanschlüsse 35a, 62a und 35b, 62b sind über eine Schlauchverbindung 6b bzw. 7b miteinander verbunden. Der Druck im Kopfraum der Probenflasche 2 wird über ein an die Druckausgleichsleitung 20 innerhalb des Ventilblockes 35 angeschlossenes Manometer 22 angezeigt.

Die Vorrichtung gemäß Figur 2 verdeutlicht insbesondere den konkreten Aufbau des Ventilblockes 35, des Gleichdruckreglers 4 und der Probenflaschenkupplung 60 in Verbindung mit der Verschlußvorrichtung 5 der Probenflasche 2. Das Abzweigrohr 31b ist als Pitotrohr mit einer in Strömungsrichtung des Prozeßmedium M orientierten Abzweigbohrung 31a ausgebildet. Des weiteren wird aus der Darstellung die konkrete Führung der Zulaufleitung 19, des ersten und des zweiten Leitungsabschnittes 16a bzw. 16b der Gasleitung 16, der Druckausgleichsleitung 20 und der Auslaßleitung 21 im Zusammenwirken mit den Ventilen 11 bzw. 12 bzw. 13 deutlich. Der Mittelschnitt durch die Probenflaschenkupplung 60 und die Verschlußvorrichtung 5 zeigen den ersten und den zweiten Verbindungsweg 6 bzw. 7 in ihrer konkreten Ausgestaltung. An dieser Stelle nicht erläuterte Bezeichnungen wurden bereits in Figur 1 abgehandelt.

Nach dem vorgeschlagenen erfindungsgemäßen Verfahren kann die über die Probenabzweiganordnung 3 mit der Durchflußleitung 1 verbundene Probenflasche 2 mit Inertgas Y gespült, vorgespannt, mit einem Probenvolumen befüllt und auf dem gleichen statischen Druck, wie er im Prozeßmedium M innerhalb der Durchflußleitung 1 vorherrscht, gehalten werden. Die Betriebszustände der erfindungsgemäßen Vorrichtung sind in den Figuren 1 und 2 mit S für Spülen, mit V für Vorspannen, mit P für Probennahme und mit A und D für den Druckausgleich gekennzeichnet. Die Kennzeichnung D steht dabei für einen Druckaufbau in der Probenflasche, wenn deren Druck unterhalb des Druckes des Prozeßmediums M liegt, und die Bezeichnung A steht für einen Druckabbau, wenn umgekehrte Druckverhältnisse vorliegen.

### Spülen S:

Das zum Spülen S erforderliche Inertgas Y wird über die Fremdgasquelle 14 bereitgestellt und strömt bei geöffneten Ventilen 12 und 13 der Probenflasche 2 in ihrem Bodenbereich über die zweite Gasleitung 16 bei entsprechend geschaltetem Zweiwege-Umschaltventil 23, den ersten Verbindungsweg 6 und das Probenabzweigrohr 53 zu. Die in der Probenflasche 2 gegebenenfalls vorhandene Luft wird dadurch über den zweiten Verbindungsweg 7 und die Auslaßleitung 21 in die Umgebung verdrängt. Nach Abschluß des Spülvorganges schließt das Ventil 13, und es kann sich der nächste Verfahrensschritt, das Vorspannen V, anschließen.

### Vorspannen V:

Die Probenflasche 2 wird auf den statischen Druck des Prozeßmediums M vorgespannt, indem Inertgas Y auf dem Weg über die zweite Gasleitung 16 einerseits über das zweite Ventil 12, die erste Schlauchverbindung 6b und das Probenabzweigrohr 53 dem Bodenbereich und andererseits über den zweiten Leitungsabschnitt 16b der Gasleitung 16, das Membrangehäuse 4a und die Druckausgleichsleitung 20 dem Kopfraum der Probenflasche 2 so lange zugeführt wird, bis Gleichdruck auf beiden Seiten der Mebrane 4b vorliegt. Gleichdruck ist dann gegeben, wenn die Membrane 4b die Abblasbohrung 4c des Gleichdruckreglers 4 so weit freigibt, daß das über den Druckmitteleintritt 4d zugeführte Inertgas Y in die Umgebung abströmen kann. Der direkte Weg der Gaseinleitung in die Probenflasche 2 über das zweite Ventil 12 parallel zum Gasweg über den Gleichdruckregler 4 ist vorgesehen, um das Vorspannen der Probenflasche 2 zügig durchzuführen. Ein Vorspannen der Probenflasche 2 ausschließlich über den Gleichdruckregler 4 dauert zu lange, da in seinem Druckmitteleintritt 4d und seinem Anschluß 4e jeweils eine Drossel angeordnet ist, die eine Zerstörung der Membrane 4b bei plötzlichem starken Druckabfall oder Druckanstieg verhindern. Die Durchtrittsquerschnitte der Drosseln sind klein gegenüber dem Querschnitt der Abblasbohrung 4c bzw. der Druckausgleichsleitung 20 ausgeführt.

### Probennahme P:

Die Probennahme P erfolgt über die Zulaufleitung 19 entweder bei kontinuierlicher oder bei getakteter Ansteuerung des ersten Ventils 11. Sie ist allein staudruckabhängig, da die statischen Drücke an der Entnahmestelle in der Durchflußleitung 1 und im Innenraum der Probenflasche über den Gleichdruckregler 4 und die Druckausgleichsleitung 19 einander angeglichen werden.

### Druckaufbau D:

Steigt der Druck des Prozeßmediums M in der Durchflußleitung 1, dann hat dies notwendigerweise auch einen Druckaufbau D in der Probenflasche 2 zur Folge. Die Membrane 4b hält bei diesen Druckverhältnissen die Abblasbohrung 4c geschlossen, so daß Inertgas Y, welches über den zweiten Leitungsabschnitt 16b der Gasleitung 16 herangeführt wird, über die Druckausgleichsleitung 20 und den zweiten Verbindungsweg 7 dem Kopfraum der Probenflasche 2 zuströmen kann. Der Druckaufbau D ist beendet, wenn die Membrane 4b beiderseits von gleichem statischen Druck beaufschlagt wird und dadurch die Abblasbohrung 4c freigibt.

### Druckabbau A:

Sinkt der Druck des Prozeßmediums M in der Durchflußleitung 1, dann muß auch zwangsläufig, damit eine staudruckabhängige Probennahme nach wie vor möglich ist, der statische Druck im Innenraum der Probenflasche 2 entsprechend abgesenkt werden. Da sich bei diesen Druckverhältnissen die Membrane 4b in Richtung des Innenraumes der Durchflußleitung 1 verformt hat, liegt die Abblasbohrung 4c in einem Maße frei, daß trotz des am Druckmitteleintritt 4d anstehenden Inertgases Y über die Druckausgleichsleitung 20 ein Druckabbau innerhalb der Probenflasche 2 über das Membrangehäuse 4a und die Abblasbohrung 4c in die Umgebung erfolgt. Bei Gleichheit der statischen Drücke in der Probenflasche 2 und im Prozeßmedium M innerhalb der Durchflußleitung 1 wird die Abblasbohrung 4c über die Membran 4b so weit gedrosselt, daß sich ein stationärer Zustand einstellen kann. Der Regelvorgang ist dann sichergestellt, wenn das über die Fremdgasquelle 14 zugeführte Inertgas Y über die Abblasbohrung 4c in die Umgebung abströmt.

Die Reinigung der Probenabzweiganordnung 3 kann erfolgen, wenn die Probenflasche 2 von ihr gelöst und Reinigungsmittel aus der Durchflußleitung 1 über die Zulaufleitung 19 in jene Bereiche abgezweigt wird, die von dem Probenstrom kontaminiert sind. Eine Sterilisation der Probenabzweiganordnung 3 erfolgt durch Dämpfen. Hierzu wird Dampf X bereitgestellt, welcher über die das Zweiwege-Umschaltventil 23 und die innerhalb des Ventilblockes 35 sich erstreckende zweite Gasleitung 16 einerseits dem ersten Verbindungsweg 6 über das zweite Ventil 12 und andererseits dem zweiten Verbindungsweg 7 über den Innenraum des Membrangehäuses 4a und die Druckausgleichsleitung 20 zuströmt. Gleichzeitig wird die Auslaßleitung 21 über das dritte Ventil 13 geöffnet. Der Dampf kann nunmehr in die Umgebung austreten. Bei der Reinigung verfährt man der Einfachheit halber ebenso, indem man das Reinigungsmittel im Wege einer sogenannten "verlorenen Reinigung" in die Umgebung abführt.

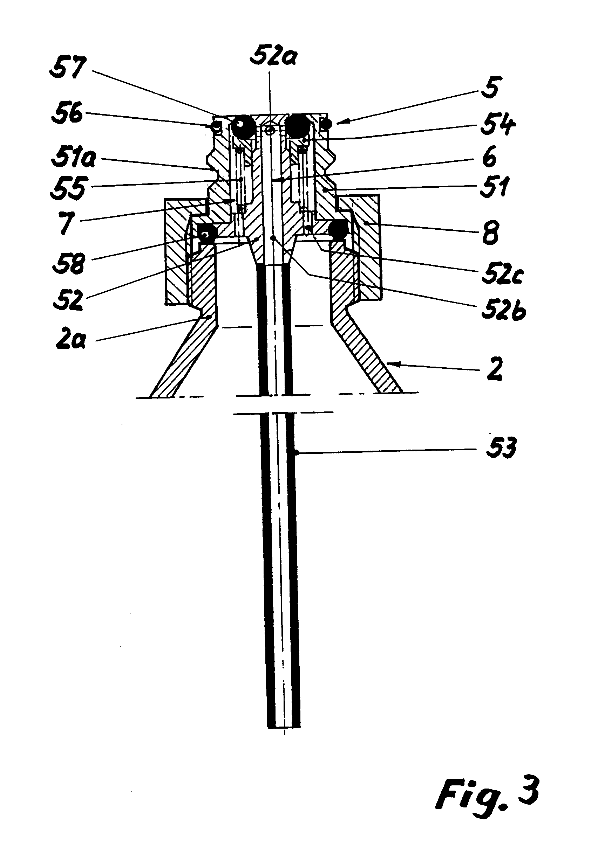

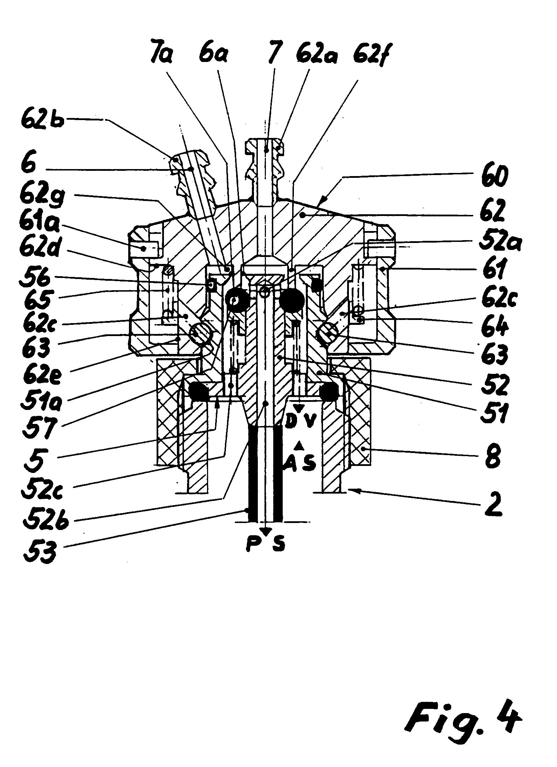

Die Probenflasche 2 (Figur 3), die vorzugsweise als druckfeste, standardisierte Glasflasche mit Schraubgewinde an einem Probenflaschenhals 2a ausgebildet ist, ist durch die Verschlußvorrichtung 5, deren prinzipieller Aufbau und Wirkungsweise an sich aus anderen Anwendungsgebieten (Verschluß von Bierfässern, US-PS 37 76 260) bekannt ist, verschlossen. Die Verschlußvorrichtung 5 besteht im einzelnen aus einem Gehäuse 51, welches an seinem Umfang im Bereich des oberen Endes eine erste Dichtung 56, an seiner Unterseite eine dritte Dichtung 58 und in seinem Mittelteil eine umlaufende Nut 51a aufnimmt. Die erste Dichtung 56 dient der Abdichtung gegenüber der Probenflaschenkupplung 60, über die Nut 51a wird eine formschlüssige Verbindung mit letzterer erreicht (siehe hierzu Figur 4), und die dritte Dichtung 58 sorgt für eine druckdichte Verbindung zur Stirnfläche des Probenflaschenhalses 2a hin. Die Verbindung des Gehäuses 51 mit der Probenflasche 2 erfolgt über ein Verbindungsteil 8, welches mit seinem Muttergewinde auf den Probenflaschenhals 2a aufschraubbar ist. Im Gehäuse 51 ist ein Mittelteil 52 angeordnet, welches eine Zentralbohrung 52b aufweist, die einerseits den Anschluß zum Probenabzweigrohr 53 und andererseits über Zulaufbohrungen 52a eine Verbindung zur Umgebung des Mittelteils 52 herstellt. Falls die Verschlußvorrichtung 5 nicht in die Probenflaschenkupplung 60 eingeführt und mit dieser verbunden ist, wie dies in Figur 3 darstellt ist, bilden das Gehäuse 51 und das Mittelteil 52 an ihrer oberen Stirnseite einen nicht bezeichneten Ringspalt, welcher über eine zweite Dichtung 57 mittels eines durch eine Feder 55 vorgespannten Verschiebeteiles 54 verschlossen wird. Der Raum zwischen dem Gehäuse 51 und dem Mittelteil 52 ist über Öffnungen 52c mit dem Innenraum der Probenflasche 2 verbunden. Die Zulaufbohrungen 52a, die sich anschließende Zentralbohrung 52b und das Probenanzweigrohr 53 bilden einen Abschnitt des ersten Verbindungsweges 6. Der Raum zwischen dem Gehäuse 51 und dem Mittelteil 52 und die Öffnungen 52c bilden einen Abschnitt des zweiten Verbindungsweges 7.

Figur 4 zeigt eine druckdicht mittels der Verschlußvorrichtung 5 verschlossene Probenflasche 2. Die Probenflaschenkupplung 60 besteht aus einer Anschlußkappe 61, die vorzugsweise auf ihrer äußeren Mantelfläche gerändelt ist, und einer federnd darin untergebrachten Kupplungshülse 62 mit den Schlauchanschlüssen 62a und 62b. Die Kupplungshülse 62 weist beiderseits jeweils einen schräg von oben nach unten verlaufenden Schlitz 62c auf, in dem jeweils ein waagerecht angeordneter Zylinderstift 63 vorgesehen ist. Die beiden Zylinderstifte 63 werden durch einen Federteller 64, der sich über eine Feder 65 an einem Bund 62d der Kupplungshülse 62 abstützt und auf einer von letzteren gebildeten zylindrischen Führung 62e gleitet, in die Nut 51a des Gehäuses 51 der Verschlußvorrichtung 5 hineingedrückt. Die Anschlußkappe 61 gleitet einerseits auf der zylindrischen Führung 62e, und sie wird andererseits auf der Kupplungshülse ein zweites mal geführt und über Anschlagstifte 61a auf der rückwärtigen Seite des Bundes 62d gegen eine nach unten gerichtete Verschiebung gesichert.

Beim Aufsetzen der Kupplungshülse 62 auf die Verschlußvorrichtung 5 wird gleichzeitig die Anschlußkappe 61 gegen den Widerstand des Federtellers 64 nach oben gedrückt, wobei jeder Zylinderstift 63 in den oberen Bereich seines Schlitzes 62c ausweichen kann. Hierdurch läßt sich die Verschlußvorrichtung 5 vollständig in die Kupplungshülse 62 bis zum Anschlag des Verbindungsteiles B an der unteren Stirnfläche der Kupplungshülse 62 hineinschieben. Nach Loslassen der Anschlußkappe 61 kann sich der Federteller 64 unter der Einwirkung der Feder 65 nach unten bewegen und drückt dabei die beiden Zylinderstifte 63 in die Nut 51a des Gehäuses 51, so daß die Probenflaschenkupplung 60 mit letzterem formschlüssig verriegelt ist. In der Endphase des Einschiebevorganges der Verschlußvorrichtung 5 in die Probenflaschenkupplung 60 greift ein ringförmiger Vorsatz 62f in den Ringspalt zwischen Gehäuse 51 und Mittelteil 52 ein und drückt dabei die zweite Dichtung 57 in Verbindung mit dem Verschiebeteil 54 gegen die Kraft der Feder 55 nach unten (siehe Figur 3), wodurch die Verbindungswege 6 und 7 geöffnet werden. Zwischen dem ringförmigen Vorsatz 62f und dem Mittelteil 52 wird innenseits ein innerer Ringspalt 6a gebildet, während zum Gehäuse 51 hin außenseits ein äußerer Durchtrittsringspalt 7a entsteht. Der erste Verbindungsweg 6 wird somit gebildet aus der Bohrung innerhalb des Schlauchanschlusses 62a und ihrer Fortsetzung innerhalb der Kupplungshülse 62, dem inneren Ringspalt 6a, den Zulaufbohrungen 52a, der Zentralbohrung 52b und dem Probenabzweigrohr 53. Der zweite Verbindungsweg 7 besteht aus der innerhalb des Schlauchanschlusses 62b gebildeten Bohrung, ihrer Fortsetzung innerhalb der Kupplungshülse 62, der Ringkammer 62g, dem äußeren Durchtrittsringspalt 7a, dem Raum zwischen dem Gehäuse 51 und dem Mittelteil 52 und den Öffnungen 52c. Die erste Dichtung 56 ist derart im Gehäuse 51 angeordnet, daß sie beim Aufsetzen der Probenflaschenkupplung 60, bevor der ringförmige Vorsatz 62f die Verbindungswege 6, 7 öffnet, eine druckfeste Verbindung zwischen dem Gehäuse 51 einerseits und der Kupplungshülse 62 andererseits herstellt. Beim Abnehmen der Probenflaschenkupplung 60 von der Verschlußvorrichtung 5 werden demzufolge die Verbindungswege 6,7 zunächst geschlossen, bevor die druckdichte Verbindung zwischen Gehäuse 51 und Kupplungshülse 62 aufgehoben wird. Druckverluste über den äußeren Durchtrittsringspalt 7a oder Flüssigkeitsverluste über den inneren Durchtrittsringspalt 6a werden damit vermieden.

Das Lösen der Probenflaschenkupplung 60 von der Verschlußvorrichtung 5 erfolgt sinngemäß in umgekehrter Reihenfolge der Handhabungsschritte, wie sie vorstehend beim Kupplungsvorgang angegeben wurden. Die Bezeichnung A, D, P, S und V bezeichnen die Betriebszustände der erfindungsgemäßen Vorrichtung, wie sie in der Beschreibung zu den Figuren 1 und 2 bereits erläutert wurden.

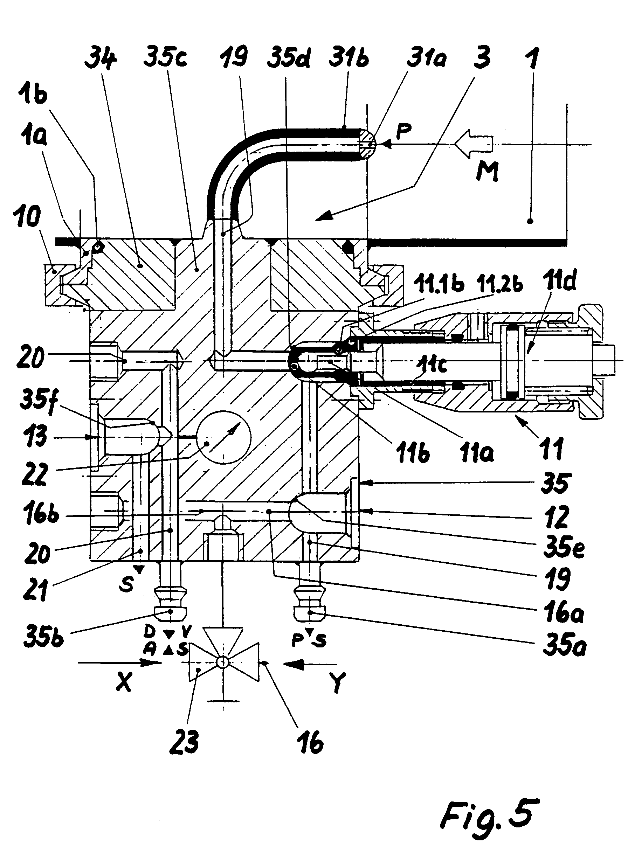

Wesentliche Merkmale des Ventilblocks 35 hinsichtlich seines Aufbaus und seiner Funktion wurden bereits in der Beschreibung zu Figur 2 genannt. Der Ventilblock 35 ist Hauptbestandteil der Probenabzweiganordnung 3 (Figur 5). Er greift mit einem Zapfen 35c in ein Anschlußteil 34 ein, schließt mit diesem am Ende des Durchgriffs bündig ab und beide sind an dieser Stelle stoffschlüssig miteinander verbunden. Das Anschlußteil 34 findet wiederum Aufnahme in einem Anschluß 1a der Durchflußleitung 1, wobei eine Gehäuse dichtung 1b für die notwendige Abdichtung sorgt. Im Zentrum des Zapfens 35c ist die Zulaufleitung 19 angeordnet, die sich innerhalb des als Pitotrohr ausgebildeten Abzweig rohres 31b bis zur Abzweigbohrung 31a fortsetzt. Die Probenabzweiganordnung 3 ist derart bündig zur inneren Begrenzungsfläche der Durchflußleitung 1 angeordnet, daß eine Beeinträchtigung der Strömung innerhalb der Durchflußleitung weitgehend ausgeschlossen ist. Die Abzweigbohrung 31a befindet sich vorzugsweise im Zentrum der Durchflußleitung 1, da Versuche gezeigt haben, daß sich Bakterien mehr im Zentrum des Querschnittes der Durchflußleitung 1 als an dessen Rand sammeln und dort daher eher aufgefunden werden können. Sie kann aber auch an anderer Stelle des Durchtrittsquerschnitts angeordnet sein, an der eine der mittleren Geschwindigkeit in der Durchflußleitung 1 entsprechende Strömungsgeschwindigkeit vorliegt. Über ein Verbindungselement 10 ist das Anschlußteil 34 mit dem Anschluß 1a der Durchflußleitung 1 formschlüssig verbunden.

Die Zulaufleitung 19 wird über das erste Ventil 11 umgeschaltet, und sie endet in einem als Schlauchanschluß 35a ausgebildeten Anschluß unterhalb des Ventilblocks 35. Die Druckausgleichsleitung 20 ist in ihrem im Ventilblock 35 angeordneten Abschnitt von keinem Ventil unterbrochen; sie verzweigt sich einerseits in die über das dritte Ventil 13 geschaltete Auslaßleitung 21 und andererseits in den Anschluß für das Manometer 22. An der Unterseite des Ventilblocks 35 endet die Druckausgleichleitung 20 in einem als Schlauchanschluß 35b ausgebildeten Anschluß. Die Gasleitung 16 ist über das Zweiwege-Umschaltventil 23 geführt und an den Ventilblock 35 angeschlossen, wo sie sich innerhalb in ihre beiden Leitungsabschnitte 16a und 16b verzweigt. Während der zweite Leitungsabschnitt 16b an seiner Austrittstelle aus dem Ventilblock 35 als Anschluß für die zum Gleichdruckregler 4 weiterführende Gasleitung ausgebildet ist, mündet der erste Leitungsabschnitt 16a in ein nicht näher bezeichnetes Gehäuse des zweiten Ventiles 12 ein. Letzteres kann wahlweise die Gasleitung 16 mit der Zulaufleitung 19 verbinden. Die mit A, D, M, P, S, V, X, Y gekennzeichneten Pfeile bezeichnen die Fließrichtung der Fluide in den vorstehend bereits erläuterten Betriebszuständen der erfindungsgemäßen Vorrichtung.

Auf die vorteilhafte Einbindung von Teilen des an sich aus der WO 90/12972 bekannten Ventiles soll anhand des ersten Ventiles 11, welches im Mittelschnitt vollständig dargestellt ist, hingewiesen werden. Die nicht dargestellten Ventile 12 und 13 sind baugleich ausgeführt und sie greifen in gleicher Weise in den Ventilblock 35 ein und wirken mit diesem zusammen. Das Ventil 11 besteht aus einem Stößel 11a, der über einen in seinem Aufbau an sich bekannten federschließenden Antrieb 11d bei Ansteuerung betätigt wird. Der Stößel 11a ist von einem haubenförmigen, elastischen Dichtungsteil 11b umschlossen, dessen offenes Ende als umlaufende Randleiste 11.2b ausgebildet und über eine Spannbüchse 11c sowohl im Ventilblock 35 als auch im angeflanschten Gehäuse des Ventils 11 eingespannt ist. Im übrigen sorgt ein Wulst 11.1b dafür, daß auch in der hier vorliegenden neuartigen Kombination zwischen Ventilblock 35 und Teilen des bekannten Ventils die in der Druckschrift WO 90/12972 zu entnehmenden Vorteile verwirklicht sind.

Die Sitzfläche 35d für das Dichtungsteil 11b ist im Ventilblock 35 ausgebildet. In gleicher Weise werden auch die Sitzflächen 35e und 35f für das zweite und das dritte Ventil 12 bzw. 13 realisiert. Auf die Vorteile, die sich mit einer derartigen Anordnung ergeben, wurde vorstehend bereits hingewiesen.

Der Ablauf des Verfahrens zur Probengewinnung und Probenhandhabung gestaltet sich kurz wie folgt:

Die Probenflasche 2 kommt mit ihrer sterilisierten Verschlußvorrichtung 5, die zweckmäßigerweise mit einer sterilen und leicht abnehmbaren Abdeckkappe geschützt ist, vom Analysenlabor und wird über die Probenflaschenkupplung 60 und deren Schlauchverbindungen 6b und 7b mit der Probenabzweiganordnung 3 verbunden (siehe Figur 2). Beim Kupplungsvorgang greift der ringförmige Vorsatz 62f der Kupplungshülse 62 in die Verschlußvorrichtung 5 ein und öffnet dadurch in der endgültigen Verbindungsstellung die beiden Verbindungswege 6 und 7 (Figur 4). Das Spülen S und das Vorspannen V der Probenflasche 2 wurden vorstehend im Zusammenhang mit der Erläuterung der Figuren 1 und 2 bereits beschrieben. Die Probennahme erfolgt über das vom Prozeßmedium M in der Durchflußleitung 1 angeströmte Abzweigrohr 31b mit seiner Abzweigbohrung 31a, welches einen Teil der Zulaufleitung 19 bildet. Letztere wird über das Schließglied 11a, 11b des ersten Ventiles 11 (Probennahmeventil) gesteuert (Figur 5). Der Probenstrom läuft ohne Stau über die Zulaufleitung 19, die Schlauchverbindung 6b, den ersten Verbindungsweg 6 und das Probenabzweigrohr 53 in den Bodenbereich der Probenflasche 2 (Figur 2).

Der Probenstrom resultiert einerseits entweder aus der vorgesehenen Taktfolge des Probennahmeventils 11 in Verbindung mit dessen Öffnungszeit oder aus dessen wahlweise veränderbaren Hub oder andererseits aus wahlweise veränderbaren Abmessungen des Durchtrittsquerschnittes im Abzweigrohr 31b oder der Zulaufleitung 19 im Bereich zwischen dem Abzweigrohr 31b und dem Probennahmeventil 11. Als Taktfolge soll der zeitliche Abstand zwischen zwei aufeinanderfolgenden Ventilöffnungen bezeichnet werden, während als Ventilöffnungszeit jene Zeit verstanden wird, die das Ventil 11 geöffnet bleibt. Die kleinstmögliche Taktfolge und die größtmögliche Ventilöffnungszeit sind so zueinander bemessen, daß sich in dieser Anwendung ein quasikontinuierlicher Probenabzweig aus der Durchflußleitung 1 ergibt.

Taktfolge und Ventilöffnungszeit werden über eine geeignete Steuerung 24 so bemessen, daß über die Zeitdauer der geplanten Probennahme die bereitgestellte Probenflasche 2 möglichst vollständig befüllt wird. Hierzu wird als Sollvorgabe die zu erwartende Probennahmezeit eingestellt. Entsprechend der Flaschengröße errechnet die Steuerung 24, zunächst unter Beibehaltung der kürzestmöglichen Ventilöffnungszeit, die notwendige Taktfolge. Ergibt sich eine Taktfolge, die kleiner ist als die minimal mögliche Taktfolge, so wird die Ventilöffnungszeit derart berechnet, daß die Probennahme mit der minimal möglichen Taktfolge gefahren werden kann. Errechnet sich bei der kleinsten Ventilöffnungszeit bereits eine Überfüllung der Probenflasche 2, so wird die Taktfolge in der notwendigen Weise verlängert. Wird im umgekehrten Fall, trotz kürzestmöglicher Taktfolge, die Probenflasche 2 nach Berechnung nicht voll, dann wird die Ventilöffnungszeit entsprechend vergrößert.

Nach Beendigung der Probennahme wird die Probenflasche 2 unter Beibehaltung des statischen Druckes aus der Probenflaschenkupplung 60 und damit von der Probenabzweiganordnung 3 gelöst und dabei druckdicht verschlossen (Figur 3). Nach Überstülpen einer sterilen Abdeckkappe über die Verschlußvorrichtung 5 kann die Probenflasche 2 transportiert und beispielsweise zum Analysenlabor gebracht werden. Mit einem aufsetzbaren Adapter, ausgestaltet ähnlich der Probenflaschenkupplung 60, wird dann die Probenflüssigkeit im Analysenlabor unter Druck und unter den selben sterilen Bedingungen wie bei der Probennahme, und zwar auf dem Weg über das Probenabzweigrohr 53, aus dem Bodenbereich der Probenflasche 2 entnommen (siehe Entleerung E in Figur 2) und in das Analysengerät überführt.

Die Reinigung und Sterilisation der Probenabzweiganordnung 3 wurde einleitend bereits prinzipiell erläutert. Die Schlauchverbindungen 6b und 7b werden von der Probenabzweiganordnung 3 entfernt und die Reinigungsflüssigkeit läuft dann bei offenem oder getaktetem Probenabzweigventil 11 über die Zulaufleitung 19 ins Freie (Figur 2). Um die Probenflasche 2 vor Überfüllung zu schützen, wird entweder im Bereich des Probenflaschenhalses 2a oder aber über in der Druckausgleichsleitung 20 ein Flüssigkeit von Gas unterscheidender Signalgeber angeordnet, über dessen diesbezügliches Signal die Probengewinnung unverzüglich unterbrochen wird. Im Bereich des Probenflaschenhalses 2a kann eine Lichtschranke, in der Druckausgleichsleitung 20 kann beispielsweise ein Grenzwertmelder in der Form einer Doppelelektrode zur Anwendung kommen. Der Bruch oder die Leckage der Probenflasche 2 oder die Leckage der Probenabzweiganordnung 3 wird zweckmäßiger Weise über einen aus diesen Ereignissen jeweils resultierenden Druckabfall erfaßt. Hierzu kann beispielsweise in der Druckausgleichsleitung 20 ein Druckgeber angeordnet werden, über dessen Signalausgang die Probengewinnung unterbrochen wird.

In gleicher Weise wird beispielsweise ein Druckabfall in der Durchflußleitung 1 erfaßt werden, so daß die beiden Verbindungswege 6, 7 zur Probenflasche 2 unverzüglich geschlossen werden können.

## Patentansprüche

1. Verfahren zur Probengewinnung und Probenhandhabung, insbesondere für Flüssigkeiten mit unter Druck gelösten Gasen im Bereich der Nahrungsmittel- und Getränkeindustrie, bei dem jeweils während der Überführung eines zu prüfenden Flüssigkeitsvolumens in einer Durchflußleitung ein dem überführten Gesamtvolumen repräsentatives Probenvolumen in vom Staudruck abhängiger Menge fortlaufend aus der Durchflußleitung in eine Probenflasche abgezweigt wird, wobei der statische Druck im abgezweigten Volumen dem statischen Druck an der Entnahmestelle der Durchflußleitung durch Regelung mittels einer Fremdgasquelle angeglichen wird, **dadurch gekennzeichnet**, daß
- eine Probenflasche (2) vorgesehen ist, die im geschlossenen, druckdichten Zustand unter sterilen Bedingungen mit einer an einer Durchflußleitung (1) angeordneten Probenabzweiganordnung (3) druckdicht verbunden wird,
- dabei ein erster Verbindungsweg (6) zwischen dem Innenraum der Probenflasche (2) und der Probenabzweiganordnung (3) der Durchflußleitung (1) und ein zweiter Verbindungsweg (7) zwischen dem Innenraum der Probenflasche (2) und einer Druckausgleichsleitung (20) geöffnet werden,
- die Druckausgleichsleitung (20) und eine mit der Fremdgasquelle (14) verbundene Gasleitung (16) mit einem Gleichdruckregler (4) verbunden werden, der durch den Druck der Durchflußleitung (1) an der Entnahmestelle beaufschlagt wird,
- das Probenvolumen zeitlich getaktet oder kontinuierlich aus der Durchflußleitung (1) mittels der Probenabzweiganordnung (3) abgezweigt und der Probenflasche (2) über den ersten Verbindungsweg (6) zugeführt wird, wobei Druckschwankungen in der Durchflußleitung (1) kontinuierlich über den Gleichdruckregler (4), die Druckausgleichsleitung (20) und den zweiten Verbindungsweg (7) im Innenraum der Probenflasche (2) ausgeglichen werden,
- die Probenflasche (2) am Ende der Probengewinnung unter Beibehaltung des statischen Druckes von der Probenabzweiganordnung (3) gelöst und dabei druckdicht verschlossen wird und daß
- das Probenvolumen im Zuge einer der Probengewinnung nachgeordneten Probenanalyse der Probenflasche (2) über den ersten Verbindungsweg (6) unter Beibehaltung des statischen Druckes und unter sterilen Bedingungen entnommen und einem Analysegerät zugeführt wird.

2. Verfahren nach Anspruch 1, **dadurch gekennzeichnet,** daß der Innenraum der Probenflasche (2) durch Zufuhr eines geeigneten Gases aus der mit der Fremdgasquelle (14) verbundenen Gasleitung (16) über die beiden Verbindungswege (6,7) durchspült und sein Inhalt durch dieses Gas substituiert wird.

3. Verfahren nach Anspruch 1 oder 2, **dadurch gekennzeichnet**, daß der Innenraum der Probenflasche (2) über die Druckausgleichsleitung (20) und den zweiten Verbindungsweg (7) mit Gas auf den am Gleichdruckregler (4) anliegenden statischen Druck an der Entnahmestelle vorgespannt wird.

4. Verfahren nach einem der Ansprüche 1 bis 3, **dadurch** **gekennzeichnet,** daß die Überfüllung der Probenflasche (2) mittels eines Flüssigkeit von Gas unterscheidenden Signalgebers erfaßt und die Probengewinnung durch ein diesbezügliches Signal unverzüglich unterbrochen wird.

5. Verfahren nach einem der Ansprüche 1 bis 3, **dadurch** **gekennzeichnet,** daß bei Bruch oder Leckage der Probenflasche (2) oder Leckage der Probenabzweiganordnung (3) ein daraus resultierender Druckabfall erfaßt und die Probengewinnung durch ein diesbezügliches Signal unverzüglich unterbrochen wird.

6. Verfahren nach einem der Ansprüche 1 bis 5, **dadurch ge****kennzeichnet,** daß bei Druckabfall in der Durchflußleitung (1) dieser erfaßt wird und die beiden Verbindungswege (6, 7) zur Probenflasche (2) unverzüglich geschlossen werden.

7. Verfahren nach einem der Ansprüche 1 bis 6, **dadurch ge****kennzeichnet,** daß die Probenflasche (2) mit einer Nährlösung oder mit einem anderen für analytische Zwecke notwendigen Substrat bzw. mit einer Lösung vorgefüllt wird, daß die Probenflasche (2) und ihr unter Abschluß stehender Inhalt gemeinsam sterilisiert werden und daß das Probenvolumen in die vorbehandelte Probenflasche (2) eingebracht wird.

8. Verfahren nach einem der Ansprüche 1 bis 7, **dadurch gekennzeichnet**, daß der Staudruck der Strömung an der Entnahmestelle in der Durchflußleitung (1) die anordnungsbedingt hinzunehmende statische Druckdifferenz zwischen der Entnahmestelle und der Austrittsstelle in der Probenflasche (2) um wenigstens das fünffache übersteigt.

9. Vorrichtung zur Durchführung des Verfahrens nach einem der Ansprüche 1 bis 8, mit einer Probenflasche, deren Innenraum über eine Zulaufleitung, die in ihrem Eintrittsbereich innerhalb der Durchflußleitung als Pitotrohr ausgebildet ist, mit der Entnahmestelle verbunden und über eine Druckausgleichsleitung mit dem statischen Druck an der Entnahmestelle beaufschlagt wird, **dadurch gekennzeichnet,** daß
- die Probenflasche (2) eine in ihrem Aufbau an sich bekannte Verschlußvorrichtung (5) aufweist, deren federschließendes Verschiebeteil (54) in Verbindung mit einer Dichtung (57) die beiden Verbindungswege (6,7) gleichzeitig schaltet,
- die Probenflasche (2) mit der an der Durchflußleitung (1) angeordneten Probenabzweiganordnung (3; 31b, 34, 35) über eine Probenflaschenkupplung (60) druckdicht verbunden wird,
- in der Verbindungsstellung zwischen Probenflasche (2) und Probenflaschenkupplung (60) ein ringförmiger Vorsatz (62f) einer Kupplungshülse (62) der Probenflaschenkupplung (60) in ein Gehäuse (51) der Verschlußvorrichtung (5) eingreift und dabei die Verbindungswege (6,7) öffnet,
- der erste Verbindungsweg (6) einen Abschnitt der Zulaufleitung (19) und der zweite Verbindungsweg (7) einen Abschnitt der Druckausgleichsleitung (20) bilden,
- sich die mit einer Fremdgasquelle (14) verbundene Gasleitung (16) in einen ersten und in einen zweiten Leitungsabschnitt (16a bzw. 16b) verzweigt und ein Gleichdruckregler (4) mit der Druckausgleichsleitung (20) und mit dem zweiten Leitungsabschnitt (16b) verbunden ist,
- die Zulaufleitung (19) über ein erstes Ventil (11) gesteuert wird,
- zwischen dem ersten Ventil (11) und der Probenflaschenkupplung (60) der durch ein zweites Ventil (12) gesteuerte erste Leigungsabschnitt (16a) in die Zulaufleitung (19) einmündet und
- die Druckausgleichsleitung (20) über eine durch ein drittes Ventil (13) gesteuerte Auslaßleitung (21) mit der Umgebung verbunden ist (Figuren 2, 3, 4, 5).

10. Vorrichtung nach Anspruch 9, **dadurch gekennzeichnet,** daß die Probenabzweiganordnung (3) einen Ventilblock (35) aufweist, an den zum einen die Ventile (11, 12 und 13) angeflanscht sind. wobei ihre Schließglieder (11a, 11b; 12a, 12b; 13a, 13b) von außen in diesen hineingreifen und dort jeweils mit einer im Ventilblock (35) ausgebildeten Sitzfläche (35d bzw, 35e bzw. 35f) zusammenwirken, und der zum anderen einen Abschnitt des zweiten Leitungsabschnittes (16b) der Gasleitung (16), einen Abschnitt der Druckausgleichsleitung (20), die Zulaufleitung (19), die Auslaßleitung (21), einen ersten Leitungsabschnitt (16a) der Gasleitung (16) und das in die Durchflußleitung (1) hineinragende Abzweigrohr (31b) aufnimmt (Figur 5).

11. Vorrichtung nach Anspruch 9 oder 10, **dadurch gekenn****zeichnet,** daß die Probenflaschenkupplung (60) als ein von der Probenabzweiganordnung (3) getrenntes, separates Bauteil ausgebildet ist.

12. Vorrichtung nach Anspruch 11, **dadurch gekennzeichnet**, daß die Zulaufleitung (19) und die Druckausgleichsleitung (20) an ihrem der Probenflaschenkupplung (60) zugewandten Ende jeweils einen Schlauchanschluß (35a bzw. 35b) aufweisen, daß die Probenflaschenkupplung (60) mit zwei Schlauchanschlüssen (62a und 62b) ausgestattet ist, von denen der erste zum ersten Verbindungsweg (6) und der zweite zum zweiten Verbindungsweg (7) führt und daß die Schlauchanschlüsse (35a und 62a) über eine Schlauchverbindung (6b) und die Schlauchanschlüsse (35b und 62b) über eine Schlauchverbindung (7b) miteinander verbunden sind.

13. Vorrichtung nach einem der Ansprüche 9 bis 12, **dadurch gekennzeichnet**, daß die Probenabzweiganordnung (3) und die Probenflaschenkupplung (60) in ihrem mit Probenflüssigkeit kontaminierten Bereich keine Dichtungen aufweisen.

14. Vorrichtung nach einem der Ansprüche 9 bis 13, **dadurch gekennzeichnet**, daß die Probenanzweiganordnung (3) in Verbindung mit der Probenflasche (2) unabhängig von der Nennweite der Durchflußleitung (1) ausgebildet ist.

15. Vorrichtung nach einem der Ansprüche 9 bis 14, **dadurch** **gekennzeichnet**, daß der Hub des ersten Ventils (11) wahlweise veränderbar ist.

16. Vorrichtung nach einem der Ansprüche 9 bis 15, **dadurch gekennzeichnet**, daß die Öffnungszeit und der zeitliche Abstand zwischen zwei Öffnungen des ersten Ventils (11) wahlweise veränderbar sind.

17. Vorrichtung nach einem der Ansprüche 9 bis 16, **dadurch** **gekennzeichnet,** daß die Zulaufleitung (19) im Bereich zwischen dem Abzweigrohr (31b) und dem ersten Ventil (11) wahlweise mit unterschiedlichen Durchtrittsquerschnitten ausgestattet wird.

18. Vorrichtung nach einem der Ansprüche 9 bis 17, **dadurch** **gekennzeichnet,** daß der erste Verbindungsweg (6) im Bodenbereich und der zweite (7) im Kopfbereich der Probenflasche (2) ausmünden.

## Claims

1. Process for obtaining and handling samples, especially for fluids with gases dissolved under pressure in the food and beverage industry, in which during the passage of a fluid volume to be tested in a through-flow line, a sample volume representative for the passed total volume is tapped continually from the through-flow line into a sample flask, in a quantity depending on the dynamic pressure, with the static pressure in the tapped volume being adapted to the static pressure at the receiving point of the through-flow line through control by means of a foreign gas source, **characterized in that**
- a sample flask (2) is provided that is connected pressure-tightly in a closed pressure-tight state under sterile conditions to a sample tapping device (3) arranged at a through-flow line (1),
- simultaneously, a first connecting path (6) between the inside of the sample flask (2) and the sample tapping device (3) of the through-flow line (1) and a second connecting path (7) between the inside of the sample flask (2) and a pressure equalizing line (20) are opened,
- the pressure equalizing line (20) and a gas line (16) connected with the foreign gas source (14) are connected with a constant-pressure control (4) that is admitted by the pressure of the through-flow line (1) at the tapping point,
- the sample volume is tapped in cycles or continually from the through-flow line (1) by means of the sample tapping device (3) and fed to the sample flask (2) over the first connecting path (6), with pressure oscillations in the through-flow line (1) being equalized continually over the constant-pressure control (4), the pressure equalizing line (20) and the second connecting path (7) in the inside of the sample flask (2),
- the sample flask (2) is separated from the sample tapping device (3) and closed pressure-tightly at the end of the sampling, maintaining the static pressure, and that
- the sample volume during an analysis of the sample flask (2) after the sampling is tapped over the first connecting path (6) under sterile conditions, maintaining the static pressure, and fed to an analysis device.

2. Process according to Claim 1, **characterized in that** the inside of the sample flask (2) is rinsed by inlet of an appropriate gas from the gas line (16) connected with the foreign gas source (14) over both connecting paths (6, 7) and its content is substituted by this gas.

3. Process according to Claim 1 or 2, **characterized in that** the inside of the sample flask (2) is prestressed at the tapping point over the pressure equalizing line (20) and the second connecting path (7) with gas to the static pressure applied at the constant-pressure control (4).

4. Process according to one of Claims 1 to 3, **characterized in that** overfilling of the sample flask (2) is registrated by means of a signal transmitter differentiating between fluid and gas, and that the sampling is interrupted immediately by an appropriate signal.

5. Process according to one of Claims 1 to 3, **characterized in that** in case of break or leakage of the sample flask (2) or leakage of the sample tapping device (3), a resulting drop in pressure is registrated and that the sampling is interrupted immediately by an appropriate signal.

6. Process according to one of Claims 1 to 5, **characterized in that** a possible drop in pressure in the through-flow line (1) is registrated, and both connecting paths (6, 7) to the sample flask (2) are closed immediately.

7. Process according to one of Claims 1 to 6, **characterized in that** the sample flask (2) is prefilled with a nourishing solution or another substrate or a solution necessary for analytical purposes, that the sample flask (2) and its shut-in content are sterilized together and that the sample volume is fed into the pretreated sample flask (2).

8. Process according to one of Claims 1 to 7, **characterized in that** the dynamic pressure of the flow at the tapping point in the through-flow line (1) exceeds at least five-fold the static pressure difference between the tapping point and the outlet point in the sample flask (2) that has to be accepted due to the arrangement.

9. Device for execution of the process according to one of Claims 1 to 8, in which the inside of the sample flask is connected with the tapping point over a feeding line that in its inlet area within the through-flow line is formed as a Pitot tube, and admitted with the static pressure at the tapping point over a pressure equalizing line, **characterized in that**
- the sample flask (2) provides a closing device (5) generally known in its arrangement, with a spring closing shunting installation (54) that in connection with a sealing (57) operates both connecting paths (6, 7) simultaneously,
- the sample flask (2) is connected pressure-tightly with the sample tapping device (3; 31b, 34, 35) arranged at the through-flow line (1) over a sample flask clutch (60),
- in the connecting position, an annular projection (62f) of a clutch sleeve (62) of the sample flask clutch (60) engages between sample flask (2) and sample flask clutch (60) into a housing (51) of the closing device (5), opening the connecting paths (6, 7),
- the first connecting path (6) forms a section of the inlet line (19) and the second connecting path (7) forms a section of the pressure equalizing line (20),
- the gas line (16) connected with a foreign gas source (14) divides into a first line section (16a) and a second line section (16b), and a constant-pressure control (4) is connected with the pressure equalizing line (20) and with the second line section (16b),
- the inlet line (19) is controlled over a first valve (11),
- between the first valve (11) and the sample flask clutch (60), the first line section (16a) controlled by a second valve (12) opens into the inlet line (19), and
- the pressure equalizing line (20) is connected with the surrounding (Figures 2, 3, 4, 5) over an outlet line (21) controlled by a third valve (13).

10. Device according to Claim 9, **characterized in that** the sample tapping device (3) provides a valve block (35) at which on one hand the valves (11, 12, and 13) are flanged, with their closing members (11a, 11b; 12a, 12b; 13a, 13b) engaging from outside into the block and cooperating with a respective seat (35d or 35e or 35f) moulded into the valve block (35) and which on the other hand receives a section of the second line section (16b) of the gas line (16), a section of the pressure equalizing line (20), the inlet line (19), the outlet line (21), a first line section (16a) of the gas line (16), and the tapping tube (31b) projecting into the through-flow line (1) (Figure 5).

11. Device according to Claim 9 or 10, **characterized in that** the sample flask clutch (60) is designed as a separate component, disconnected from the sample tapping device (3).

12. Device according to Claim 11, **characterized in that** the inlet line (19) and the pressure equalizing line (20) are provided at their respective end towards the sample flask clutch (60) with a tube coupling (35a and 35b), that the sample flask clutch (60) is provided with two tube couplings (62a and 62b), the first one leading to the first connecting path (6) and the second one leading to the second connecting path (7), and that the tube couplings (35a and 62a) are connected with each other over a tube connection (6b) and the tube couplings (35b and 62b) over a tube connection (7b).

13. Device according to one of Claims 9 to 12, **characterized in that** the sample tapping device (3) and the sample flask clutch (60) provide no sealings in their areas contaminated with sample fluid.

14. Device according to one of Claims 9 to 13, **characterized in that** the sample tapping device (3) in connection with the sample flask (2) is formed independently from the nominal width of the through-flow line (1).

15. Device according to one of Claims 9 to 14, **characterized in that** the stroke of the first valve (11) can be changed optionally.

16. Device according ot one of Claims 9 to 15, **characterized in that** the opening time and the period between two openings of the first valve (11) can be changed optionally.

17. Device according to one of Claims 9 to 16, **characterized in that** the inlet line (19) is provided optionally with different cross-sectional areas between the tapping tube (31b) and the first valve (11).

18. Device according to one of Claims 9 to 17, **characterized in that** the first connecting path (6) opens out in the bottom part and the second one in the top part of the sample flask (2).

## Revendications

1. Procédé pour l'extraction d'échantillons et la manutention d'échantillons, en particulier pour les liquides comportant des gaz dissous sous pression dans le domaine de l'industrie alimentaire et des boissons, selon lequel, à chaque transfert d'un volume de liquide à contrôler dans une conduite de passage, un volume d'échantillon représentatif du volume total transféré, dont la quantité dépend de la pression dynamique, est dérivé de façon continue de la conduite de passage et amené dans une bouteille d'échantillonnage, la pression statique dans le volume dérivé étant adaptée à la pression statique à l'endroit de prélèvement dans la conduite de passage par réglage au moyen d'une source de gaz étranger, caractérisé en ce que
- il est prévu une bouteille d'échantillonnage (2) qui, en état fermé et étanche et sous conditions aseptiques est reliée de manière étanche à un réseau de dérivation d'échantillons (3) disposé sur une conduite de passage (1),
- on ouvre un premier chemin de liaison (6) entre l'intérieur de la bouteille d'échantillonnage (2) et le réseau de dérivation d'échantillons (3) de la conduite de passage (1), et un deuxième chemin de liaison (7) entre l'intérieur de la bouteille d'échantillonnage (2) et une conduite d'égalisation de pression (20),
- la conduite d'égalisation de pression (20) et une conduite de gaz (16) reliée à la source de gaz étranger (14) sont reliées à un équilibreur (4) qui est commandé par la pression de la conduite de passage (1) à l'endroit de prélèvement,
- le volume d'échantillon est dérivé à intervalles réguliers ou en continu de la conduite de passage (1) au moyen du réseau de dérivation d'échantillons (3) et amené à la bouteille d'échantillonnage (2) à travers le chemin de liaison (6), les fluctuations de pression dans la conduite de passage (1) étant équilibrées en continu par l'équilibreur (4), la conduite d'égalisation de pression (20) et le deuxième chemin de liaison (7) à l'intérieur de la bouteille d'échantillonnage (2),
- la bouteille d'échantillonnage (2) est retirée du réseau de dérivation d'échantillons (3) à la fin de l'extraction d'échantillons en maintenant la pression statique et en la fermant de façon étanche, et que
- le volume d'échantillon, au cours d'une analyse d'échantillon ayant lieu après l'extraction, est prélevé de la bouteille d'échantillonnage (2) à travers le premier chemin de liaison (6) en maintenant la pression statique et sous conditions aseptiques et amené à un appareil d'analyse.

2. Procédé selon la revendication 1, caractérisé en ce que l'intérieur de la bouteille d'échantillonnage (2) est rincé par l'amenée d'un gaz adéquat provenant de la conduite de gaz (16) reliée à la source de gaz étranger (14) à travers les deux chemins de liaison (6, 7) et que son contenu est substitué par ce gaz.

3. Procédé selon l'une des revendications 1 ou 2, caractérisé en ce que l'intérieur de la bouteille d'échantillonnage (2) est précontraint à travers la conduite d'égalisation de pression (20) et le deuxième chemin de liaison (7) avec du gaz sur la pression statique adjacente à l'équilibreur à l'endroit de prélèvement.

4. Procédé selon l'une des revendications 1 à 3, caractérisé en ce que le remplissage excessif de la bouteille d'échantillonnage (2) est détecté au moyen d'un poste transmetteur de signaux faisant la différence entre le liquide et le gaz, et que l'extraction d'échantillons est immédiatement interrompue par un signal correspondant.

5. Procédé selon l'une des revendications 1 à 3, caractérisé en ce qu'en cas de casse ou de fuite de la bouteille d'échantillonnage (2) ou de fuite du réseau de dérivation d'échantillons (3), une chute de pression qui en résulte est détectée et que l'extraction d'échantillons est immédiatement interrompue par un signal correspondant.

6. Procédé selon l'une des revendications 1 à 5, caractérisé en ce que, en cas de chute de pression dans la conduite de passage (1), celle-ci est détectée et les deux chemins de liaison (6, 7) menant à la bouteille d'échantillonnage (2) sont immédiatement fermés.

7. Procédé selon l'une des revendications 1 à 6, caractérisé en ce que la bouteille d'échantillonnage (2) est remplie auparavant avec un bouillon de culture ou un autre substrat nécessaire à des fins analytiques ou avec une solution, que la bouteille d'échantillonnage (2) et son contenu enfermé sont stérilisés ensemble et que le volume d'échantillon est introduit dans la bouteille d'échantillonnage (2) préparée.

8. Procédé selon l'une quelconque des revendications 1 à 7, caractérisé en ce que la pression dynamique de l'écoulement à l'endroit de prélèvement dans la conduite de passage (1) est au moins cinq fois plus élevée que la différence de pression statique due à la disposition entre l'endroit de prélèvement et l'endroit de sortie dans la bouteille d'échantillonnage (2).

9. Procédé pour la mise en oeuvre du procédé selon l'une quelconque des revendications 1 à 8, comportant une bouteille d'échantillonnage dont l'intérieur est relié à l'endroit de prélèvement à travers une conduite d'amenée qui, dans sa zone d'entrée à l'intérieur de la conduite de passage, est concue comme un tube de Pitot et alimentée en pression statique à l'endroit de prélèvement à travers une conduite d'égalisation de pression, caractérisé en ce que
- la bouteille d'échantillonnage (2) présente un dispositif de fermeture (5) de construction en principe connue, dont la pièce coulissante (54) fermant par ressort, commute simultanément, en liaison avec un joint, (57) les deux chemins de liaison (6, 7)
- la bouteille d'échantillonnage (2) est reliée de façon étanche au réseau de dérivation d'échantillons (3; 31b, 34, 35) placé sur la conduite de passage à travers un dispositif d'accouplement (60) de la bouteille d'échantillonnage,
- dans la position de liaison entre la bouteille d'échantillonnage (2) et le dispositif d'accouplement (60) de la bouteille d'échantillonnage, une saillie en forme d'anneau (62f) d'un fourreau d'accouplement (62) du dispositif d'accouplement (60) de la bouteille d'échantillonnage vient en prise dans un boîtier (51) du dispositif de fermeture (5) et ouvre ainsi les chemins de liaison (6, 7),
- le premier chemin de liaison (6) forme un tronçon de la conduite d'amenée (19) et le deuxième chemin de liaison (7) un tronçon de la conduite d'égalisation de pression (20),
- la conduite de gaz (16) reliée à une source de gaz étranger (14) se ramifie en un premier et un deuxième tronçon de conduite (16a ou 16b) et qu'un équilibreur (4) est relié à la conduite d'égalisation de pression (20) et au deuxième tronçon de conduite (16b),
- la conduite d'amenée (19) est commandée par une première soupape (11),
- le premier tronçon de conduite (16a), commandé par une deuxième soupape (12), débouche dans la conduite d'amenée (19) entre la première soupape (11) et le dispositif d'accouplement (60) de la bouteille d'échantillonnage, et
- la conduite d'égalisation de pression (20) est reliée à la périphérie à travers une conduite d'échappement (21) commandée par une troisième soupape (13) (figures 2, 3, 4, 5).

10. Dispositif selon la revendication 9, caractérisé en ce que le réseau de dérivation d'échantillons (3) présente un bloc de soupapes (35) auquel, d'une part, sont fixées par bride les soupapes (11, 12 et 13) dont les éléments de fermeture (11a, 11b; 12a, 12b; 13a, 13b) viennent en prise de l'extérieur dans celui-ci et y coopèrent avec respectivement une surface de siège (35d ou 35e ou 35f) formée dans le bloc de soupapes (35), et, qui, d'autre part, reçoit un tronçon du deuxième tronçon de conduite (16b) de la conduite de gaz (16), un tronçon de la conduite d'égalisation de pression (20), la conduite d'amenée (19), la conduite d'échappement (21), un premier tronçon de conduite (16a) de la conduite de gaz (16) et le tube de dérivation (31b) débouchant dans la conduite de passage (1) (figure 5).

11. Dispositif selon l'une des revendications 9 ou 10, caractérisé en ce que le dispositif d'accouplement de la bouteille d'échantillonnage (60) se présente sous forme d'un élément de construction isolé, séparé du réseau de dérivation d'échantillons (3).

12. Dispositif selon la revendication 11, caractérisé en ce que la conduite d'amenée (19) et la conduite d'égalisation de pression (20) présentent, à leur extrémité tournée vers le dispositif d'accouplement (60) de la bouteille d'échantillonnage, respectivement un raccord de tuyau flexible (35a et 35b), que le dispositif d'accouplement (60) de la bouteille d'échantillonnage est pourvu de deux raccords de tuyaux flexibles (62a et 62b) dont le premier conduit au premier chemin de liaison (6) et le second au second chemin de liaison (7) et que les raccords de tuyau flexible (35a et 62a) sont reliés entre eux par un tuyau flexible (6b), et les raccords de tuyau flexible (35b et 62 b) par un tuyau flexible (7b).

13. Dispositif selon l'une des revendications 9 à 12, caractérisé en ce que le réseau de dérivation d'échantillons (3) et le dispositif d'accouplement (60) de la bouteille d'échantillonnage ne présentent pas de joints dans leur partie contaminée par le liquide prélevé.

14. Dispositif selon l'une des revendications 9 à 13, caractérisé en ce que le réseau de dérivation d'échantillons (3) en liaison avec la bouteille d'échantillonnage (2) est conçu indépendamment du diamètre nominal de la conduite de passage (1).

15. Dispositif selon l'une quelconque des revendications 9 à 14, caractérisé en ce que la course de la première soupape (11) peut être modifiée à volonté.

16. Dispositif selon l'une des revendications 9 à 15, caractérisé en ce que le temps d'ouverture et l'intervalle de temps entre deux ouvertures de la première soupape (11) peuvent être modifiés à volonté.

17. Dispositif selon l'une quelconque des revendications 9 à 16, caractérisé en ce que la conduite d'amenée (19) dans la partie entre le tube de dérivation (31b) et la première soupape (11) est prévue à volonté avec des sections de passage différentes.

18. Dispositif selon l'une des revendications 9 à 17, caractérisé en ce que le premier chemin de liaison (6) débouche au niveau du fond, et le deuxième (7) au niveau de la tête de la bouteille d'échantillonnage (2).
